(19) Europäisches Patentamt
European Patent Office
Office européen des brevets

(11) Veröffentlichungsnummer: **0 246 551 A2**

(12) # EUROPÄISCHE PATENTANMELDUNG

(21) Anmeldenummer: **87106916.7**

(51) Int. Cl.4: **C07D 263/62** , C08K 5/35

(22) Anmeldetag: **13.05.87**

(30) Priorität: **23.05.86 DE 3617451**

(43) Veröffentlichungstag der Anmeldung:
**25.11.87 Patentblatt 87/48**

(84) Benannte Vertragsstaaten:
**CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Schinzel, Erich, Dr.**
**Ostpreussenstrasse 43**
**D-6238 Hofheim am Taunus(DE)**
Erfinder: **Frischkorn, Hans, Dr.**
**Martin-Wohmann Strasse 3**
**D-6238 Hofheim am Taunus(DE)**
Erfinder: **Martini, Thomas, Dr.**
**Odenwaldstrasse 5**
**D-6233 Kelkheim (Taunus)(DE)**

(54) **Sulfonat- oder Sulfonamidgruppen-haltige Bis-benzoxazolylnaphthaline, Verfahren zu deren Herstellung und deren Verwendung.**

(57) Verbindungen der allgemeinen Formel

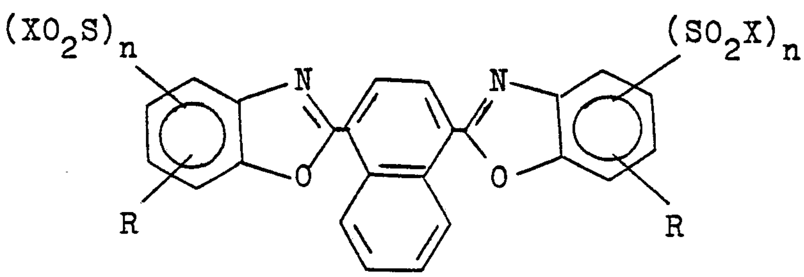

(1)

in welcher
n 1 oder 2,
X eine OMe-Gruppe,
Me ein Proton, ein Alkalimetallkation oder ein gegebenenfalls substituiertes Ammoniumkation,
R Wasserstoff, eine niedere Alkylgruppe oder Halogen und für den Fall, daß
n gleich 1 ist,
X auch eine $-NR^1R^2$-Gruppe, wobei $R^1$ und $R^2$ für Wasserstoff oder eine niedere Alkylgruppe steht, bedeuten,

Verfahren zu deren Herstellung und deren Verwendung als Optische Aufheller, insbesondere für Polyamidfasern und Wolle.

EP 0 246 551 A2

# Sulfonat-oder Sulfonamidgruppen-haltige Bis-benzoxazolylnaphthaline, Verfahren zu deren Herstellung und deren Verwendung

Es ist bereits bekannt, Sulfonatgruppen-haltige Bis-benzoxazolylnaphthaline, welche eine Sulfonatgruppe im Naphthalinkern enthalten, als Optische Aufheller zu verwenden. So wird in der DE-OS 2 645 301 z.B. das 1,4-Bis-[benzoxazolyl-(2)]-naphthalin-6-dimethylammoniumsulfonat als Optischer Aufheller beschrieben.

Es wurde nun gefunden, daß Verbindungen der allgemeinen Formel (1),

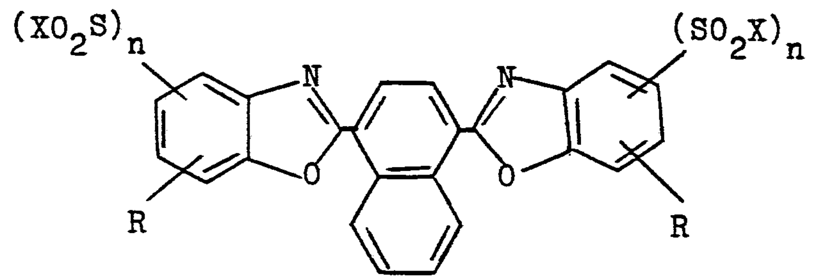

(1)

in welcher
n 1 oder 2,
X eine OMe-Gruppe,
Me ein Proton, ein Alkalimetallkation oder ein gegebenenfalls substituiertes Ammoniumkation,
R Wasserstoff, eine niedere Alkylgruppe oder Halogen und für den Fall, daß
n gleich 1 ist,
X auch eine $-NR^1R^2$-Gruppe, wobei $R^1$ und $R^2$ für Wasserstoff oder eine niedere Alkylgruppe steht, bedeuten,
sehr gute Optische Aufheller sind.

Bevorzugt sind Verbindungen der Formel (1),
bei welchen
n gleich 1,
X eine OMe-oder $NH_2$-Gruppe,
Me ein Proton, ein Alkalimetallkation oder ein gegebenenfalls substituiertes Ammoniumkation und
R Wasserstoff bedeuten.

Von ganz besonderem Interesse sind die Verbindungen der Formel (2),

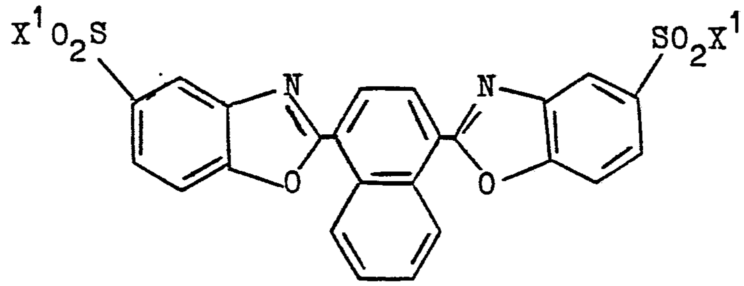

(2)

in welcher
$X^1$ eine OMe-oder $NH_2$-Gruppe und
Me ein Proton, ein Alkalimetallkation oder ein gegebenenfalls substituiertes Ammoniumkation, bedeuten.

In allen Fällen enthalten niedere Alkylgruppen 1 bis 6, vorzugsweise 1 bis 4 C-Atome. Als gegebenenfalls substituierte Ammoniumkationen kommen vor allem die Ammoniumionen in Frage, die sich von $C_1$-$C_4$-mono-, di-oder tri-Alkylamin oder mono-, di-oder tri-Hydroxyethyl-oder Hydroxypropyl-amin ableiten. Halogen bedeutet vorzugsweise Chlor oder Brom.

Die Herstellung der Verbindungen der Formel (1), bei welchen X eine OMe-Gruppe bedeutet, kann durch die in folgender Gleichung, wobei n und R die eingangs angegebene Bedeutung besitzen, ausgedrückte Kondensation in Polyphosphorsäure erfolgen.

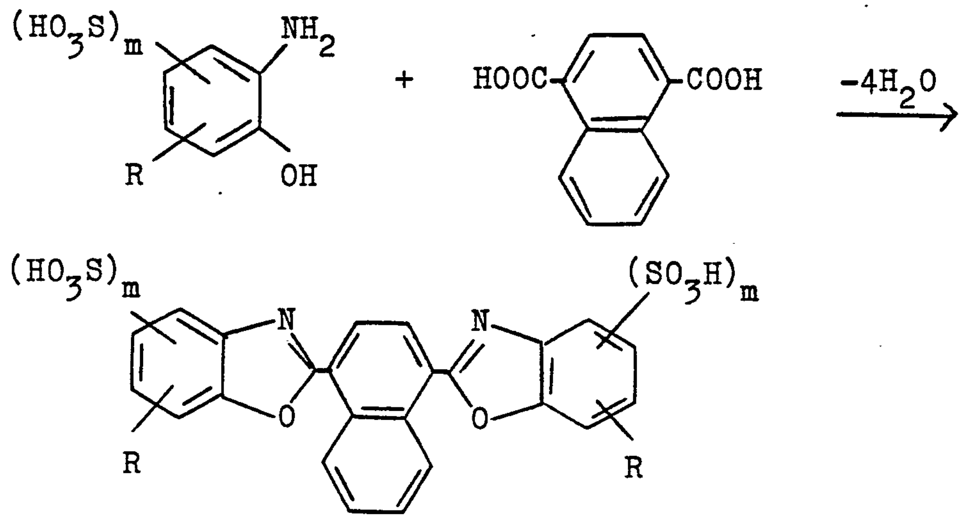


Die so erhaltenen Aufhellersulfosäuren können durch Neutralisation in die entsprechenden, durch die allgemeine Formel (1) umfaßten Salze, umgewandelt werden. Diese Kondensation wird bei 120 bis 200°C, bevorzugt 140 bis 180°C durchgeführt.

Die Sulfonamide der allgemeinen Formel (1) können, gemäß der nachstehenden Reaktionsgleichung durch Umsetzung des Säurechlorids der Naphthalin-1,4-dicarbonsäure mit entsprechenden o-Amino-phenolsulfonamiden und anschließende Wasserabspaltung hergestellt werden.

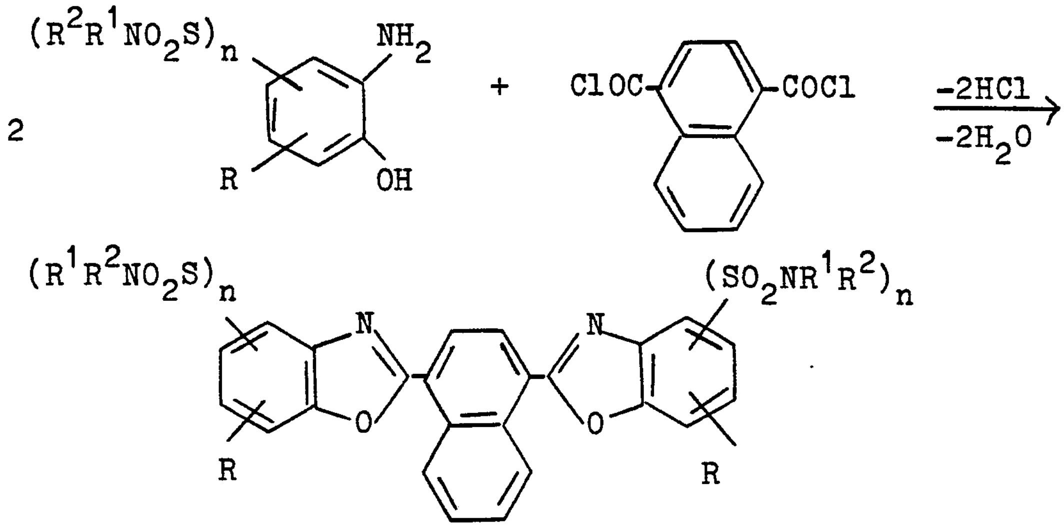


Die vorstehende Reaktion kann mit oder ohne Isolierung der als Zwischenstufe auftretenden Acylaminoverbindung

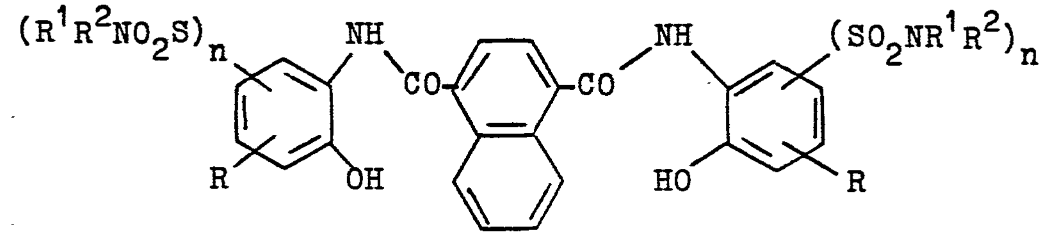

durchgeführt werden. Als Lösungsmittel für die Acylierung können aprotische Lösungsmittel wie Dichlorbenzol, Trichlorbenzol, Benzoesäuremethylester, Tetralin, N-Methyl-pyrrolidon und Dimethylformamid eingesetzt werden. Die Wasserabspaltung erfolgt bevorzugt in Gegenwart saurer Katalysatoren wie Zinkchlorid, p-Toluolsulfonsäure, N-Methyl-pyrrolidon Hydrochlorid oder Borsäure. Als Temperatur für die Benzoxazolbildung werden 140 bis 210°C angewendet. Bei der Wahl geeigneter Lösungsmittel kann das vorstehende Verfahren auch zur Herstellung der erfindungsgemäßen Aufhellerdisulfonsäuren eingesetzt werden.

Die erfindungsgemäßen Sulfonamide können auch durch Umwandlung der Aufhellersulfonsäuren in die entsprechenden Sulfochloride und anschließende Umsetzung mit Aminen der Formel $HNR^1R^2$ hergestellt werden. Zur Sulfochloridherstellung sind die üblichen Reagenzien wie Thionylchlorid, Phosphortri-und Phosphorpentachlorid geeignet.

Als o-Amino-phenol-sulfonsäuren und -sulfonamide können beispielsweise eingesetzt werden:

2-Amino-phenol-4-sulfonsäure
2-Amino-phenol-5-sulfonsäure
4-Methyl-2-amino-phenol-6-sulfonsäure
6-Methyl-2-amino-phenol-4-sulfonsäure
4-Chlor-2-amino-phenol-6-sulfonsäure
6-Chlor-2-amino-phenol-4-sulfonsäure
2-Amino-phenol-4,6-disulfonsäure
2-Amino-phenol-4-sulfonamid
2-Amino-phenol-5-sulfonamid

Die erfindungsgemäßen neuen Verbindungen zeigen in gelöstem oder feinverteiltem Zustande eine mehr oder weniger ausgeprägte Fluoreszenz. Sie werden zum optischen Aufhellen der verschiedensten synthetischen, halbsynthetischen oder natürlichen organischen Materialien verwendet.

Hierfür seien beispielsweise, ohne daß durch die nachfolgende Übersicht irgendeine Beschränkung hierauf ausgedrückt werden soll, die folgenden Gruppen von organischen Materialien genannt:

1. Polymerisationsprodukte, beispielsweise Polymerisate auf Basis von $\alpha,\beta$-ungesättigten Carbonsäuren oder Derivaten solcher Carbonsäuren, insbesondere von Acrylverbindungen, von Olefin-Kohlenwasserstoffen und Polymerisate auf Basis von Vinyl-und Vinyliden-Verbindungen.

2. Polymerisationsprodukte die durch Ringöffnung erhältlich sind, z.B. Polyamide vom Polycaprolactam-Typ, ferner Polymere, die sowohl über Polyaddition als auch Polykondensation erhältlich sind, wie Polyether oder Polyacetale.

3. Polykondensationsprodukte oder Vorkondensate auf Basis di-oder polyfunktioneller Verbindungen mit kondensationsfähigen Gruppen, deren Homo-und Mischkondensationsprodukte, wie beispielsweise Polyester, insbesondere gesättigte (z.B. Ethylenglykolterephthalsäure-Polyester), unverzweigte sowie verzweigte (auch auf Basis höherwertiger Alkohole, wie z.B. Alkydharze) Polyester, Polyamide (z.B. Hexamethylendiamin-adipat).

4. Halbsynthetische organische Materialien, z.B. Celluloseester verschiedener Veresterungsgrade (sogenanntes 2 1/2-Acetat, Triacetat), Celluloseether oder regenerierte Celullose.

5. Natürliche organische Materialien animalischen oder vegetabilischen Ursprungs, beispielsweise auf Basis von Cellulose oder Proteinen wie Baumwolle, Wolle, Leinen und Seide.

Bevorzugte organische Materialien sind solche aus Polyamid und Wolle.

Die optisch aufzuhellenden organischen Materialien können den verschiedenartigsten Verarbeitungszuständen (Rohstoffe, Halbfabrikate oder Fertigfabrikate) angehören.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermassen vorliegen.

Den erfindungsgemäß anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strängen, Geweben, Gewirken, Vliesen, beflockten Substraten oder Verbundstoffen vorliegen können, erfindungsgemäß optisch aufzuhellen sind, geschieht dies mit Vorteil in wässerigem Medium, worin die betreffenden Verbindungen gelöst oder in feinverteilter Form (Suspensionen, sogenannten Mikrodispersionen) vorliegen. Gegebenenfalls können bei der Behandlung Dispergier-, Stabilisier-, Netz-und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140°C, beispielsweise bei Siedetemperatur des Bades oder in deren Nähe (etwa 90°C), durchgeführt. Für die erfindungsgemäße Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgußmasse beifügen.

Die neuen optischen Aufhellmittel gemäß vorliegender Erfindung können beispielsweise auch in folgenden Anwendungsformen eingesetzt werden:

1. Mischungen mit Farbstoffen (Nuancierung) oder Pigmenten oder als Zusatz zu Färbebädern, Druck-, Ätz-oder Reservepasten. Ferner auch zur Nachbehandlung von Färbungen, Drucken oder Ätzdrucken,

2. in Mischungen mit sogenannten "Carriern", Netzmitteln, Weichmachern, Quellmitteln, Antioxydantien, Lichtschutzmitteln, Hitzestabilisatoren und besonders chemischen Bleichmitteln (Chlorit-Bleiche, Bleichbäder-Zusätze),

3. in Mischung mit Vernetzern, Appreturmitteln (z.B. Stärke oder synthetischen Appreturen) sowie in Kombination mit den verschiedensten Textilveredlungsverfahren, insbesondere Kunstharzausrüstungen, ferner Flammfest-, Weich griff-, Schmutzablöse oder Antistatisch-Ausrüstungen oder antimikrobiellen Ausrüstungen,

4. in Kombination mit anderen, optisch aufhellend wirkenden Substanzen.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemäßen Aufhellern zweckmäßig in der Weise, daß man diese Fasern mit den wässerigen Dispersionen (gegebenenfalls auch Lösungen) der Aufhellmittel bei Temperaturen unter 75°C, z.B. bei Raumtemperatur imprägniert und einer trockenen Wärmebehandlung bei Temperaturen über 100°C unterwirft, wobei es sich im allgemeinen empfiehlt, das Fasermaterial vorher noch bei mäßig erhöhter Temperatur, z.B. bei mindestens 60°C bis etwa 130°C zu trocknen. Die Wärmebehandlung in trockenem Zustande erfolgt dann vorteilhaft bei Temperaturen zwischen 120 und 225°C, beispielsweise durch Erwärmen in einer Trockenkammer, durch Bügeln im angegebenen Temperaturintervall oder auch durch Behandeln mit trockenem, überhitztem Wasserdampf.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Für die meisten praktischen Belange sind vorzugsweise Mengen zwischen 0,01 und 0,5 Gewichtsprozent von Interesse.

Die erfindungsgemäßen Aufheller können auch in Mischung mit anderen Aufhellern eingesetzt werden. Da einige der erfindungsgemäßen Aufheller Aufhelleffekte mit grüner Nuance bewirken, ist es besonders vorteilhaft, sie gemeinsam mit Aufhellern zu verwenden, die rotstichige Aufhelleffekte zeigen.

Die neuen optischen Aufheller haben den besonderen Vorteil, daß sie auch bei Gegenwart von Aktivchlorspendern, wie z.B. Hypochlorit, wirksam sind und ohne wesentliche Einbuße der Effekte in Waschbädern mit nichtionogenen Waschmitteln, z.B. Alkylphenolpolyglykoläthern, verwendet werden können.

In den Beispielen sind Teile, soweit nicht anders angegeben, immer Gewichtsteile und Prozente immer Gewichtsprozente. Schmelz-und Siedepunkte sind, sofern nicht anders vermerkt, unkorrigiert.

Beispiel 1

54,05 g Naphthalin-1,4-dicarbonsäure werden in Mischung mit 99,35 g 2-Amino-phenol-4-sulfonsäure (100 %ig gerechnet) bei 80 bis 100°C in 750 g Polyphosphorsäure eingetragen, das Reaktionsgemisch auf 150 bis 160°C aufgeheizt und 4 Stunden bei dieser Temperatur verrührt. Nach Abkühlen auf 100°C gießt man das Reaktionsgemisch auf 1500 ml Wasser und stellt mit konzentrierter Natronlauge (ca. 1800 g, 33 %ig) neutral. Man erhitzt nochmals kurze Zeit zum Sieden, saugt bei Raumtemperatur ab und wäscht mit 10 %iger Salzlösung nach. Nach Trocknen bei 100°C im Vakuum erhält man 155 g salzhaltiges hellgelbes Produkt, das z.B. durch Umkristallisation aus wäßrigem Dimethylformamid weiter gereinigt werden kann. Das erhaltene Dinatriumsalz des 1,4-Bis-[5-sulfobenzoxazolyl-(2)]-naphthalins (11) schmilzt bis 360°C nicht.

Beispiel 2

54,05 g Naphthalin-1,4-dicarbonsäure werden in 300 ml N-Methylpyrrolidon suspendiert, bei 10 bis 15°C mit 52 g Thionylchlorid langsam versetzt und 1 Stunde bei Raumtemperatur nachgerührt. Anschließend trägt man 99,35 g 2-Amino-phenol-4-sulfonsäure (100 %ig gerechnet) ein, heizt innerhalb von ca. 1 bis 2 Stunden auf 150°C und rührt ca. 3 Stunden bei dieser Temperatur nach. Nach Abkühlen auf 100°C gießt man das Reaktionsgemisch auf 1200 ml Wasser, stellt mit konzentrierter Natronlauge alkalisch, gibt 150 g Natriumchlorid hinzu und erhitzt 30 Minuten zum Sieden. Nach Abkühlen auf Raumtemperatur saugt man ab und wäscht mit 10 %iger Salzlösung nach. Nach Trocknen bei 100°C erhält man ca. 110 g salzhaltiges dunkelgelbes Produkt. Nach Umkristallisation aus wäßrigem Dimethylformamid oder Lösen in Wasser und Ausfällen mit Aceton erhält man die Verbindung (11) in reiner Form.

Beispiel 3

54,05 g Naphthalin-1,4-dicarbonsäure werden in 200 ml Toluol unter Zusatz von 0,2 ml Pyridin suspendiert. Man heizt auf 70°C auf, setzt 52 g Thionylchlorid zu und heizt weiter innerhalb von 3 Stunden auf 110°C. Es entsteht eine Lösung. Anschließend destilliert man das Lösungsmittel vollständig ab und nimmt das zurückgebliebene Säurechlorid mit 200 ml trockenem Aceton auf. Die entstandene acetonische Lösung läßt man zu einer Suspension von 79 g 2-Amino-phenol-4-sulfonsäureamid in 200 ml trockenem Aceton und 48 g Dimethylanilin zulaufen und erhitzt 2 Stunden am Rückfluß. Nach Abkühlen verdünnt man mit 400 ml Wasser, säuert mit 23 g konzentrierter Salzsäure an, erhitzt nochmals eine halbe Stunde am Rückfluß, saugt kalt ab und wäscht mit Wasser neutral. Nach dem Trocknen im Vakuum bei 100°C erhält man die Acylaminoverbindung in einer Ausbeute von 108,5 g vom Schmp. 239 - 240°C.

Die erhaltene Acylaminoverbindung wird in 500 ml 1,2,4-Trichlorbenzol unter Zusatz von 3 g p-Toluolsulfonsäure unter Stickstoff 3 Stunden auf 210°C erhitzt. Nach Abkühlen auf Raumtemperatur wird abgesaugt und mit Methanol nachgewaschen. Das feuchte Nutschgut wird nochmals mit verdünnter Ammoniumhydroxidlösung ausgerührt und erneut isoliert, mit Wasser neutral gewaschen und im Vakuum bei 100°C getrocknet. Man erhält 71,0 g gelbbraunes Produkt. Nach Umkristallisation aus Ethylenglykol schmilzt das gelbe 1,4-Bis-[5-sulfamoyl-benzoxazolyl-(2)]-naphthalin (18) bei 316 bis 318°C.

In analoger Weise, wie in den Beispielen 1 bis 2 angegeben, können auch die in der folgenden Tabelle angegebenen Verbindungen (12) bis (17) hergestellt werden.

Tabelle 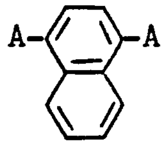

| Lfd. Nr. | A | Absorption a) $\lambda_{max}$ [nm] | Absorption a) $\varepsilon \cdot 10^{-4}$ | Fluoreszenz a) $\lambda_{max}$ [nm] | Fluoreszenz a) Q b) |
|---|---|---|---|---|---|
| (11) | 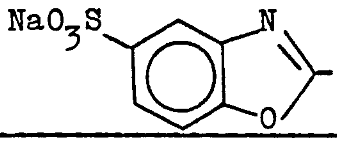 | 365 | 3,16 | 441 | 0,81 |
| (12) | 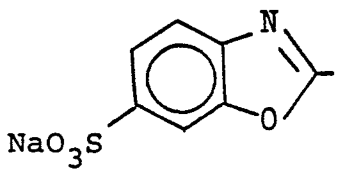 | 368 | 3,47 | 446 | 0,84 |
| (13) | 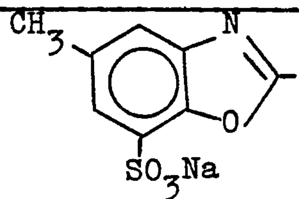 | 372 | 3,27 | 447 | 0,88 |
| (14) | 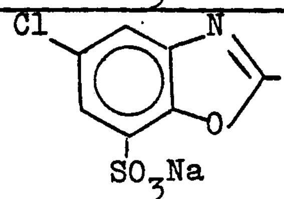 | 374 | 3,22 | 447 | 0,82 |
| (15) | 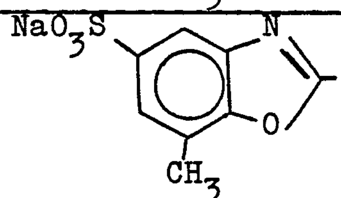 | 365 | 3,17 | 443 | 0,80 |
| (16) | 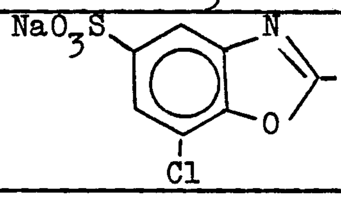 | 364 | 2,89 | 443 | 0,76 |
| (17) | 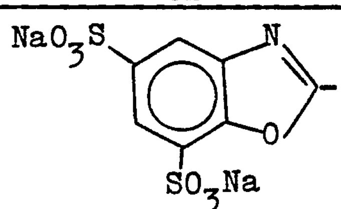 | 373 | 2,93 | 444 | 0,86 |
| (18) | 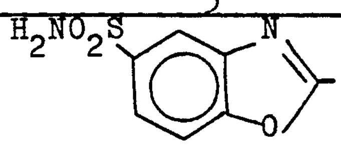 | 374 c) | 3,50 | 438 c) | 0,71 |

a) Vermessen in DMF/Wasser (3:2)
b) Quantenausbeute
c) Vermessen in DMF

Anwendungsbeispiele

Beispiel 4

20 g Polyamid-6-Taft wurden in einem Labor-Baumfärbeapparat aufgehellt. Verwendet wurde die wasserlösliche Verbindung aus Beispiel 1 der Formel

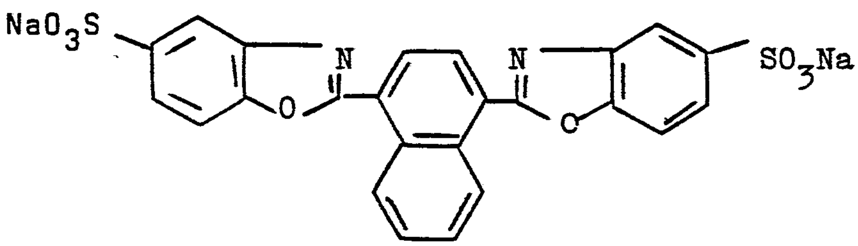

Flottenzusammensetzung:

Flotte 1:20,
0,25 % Optischer Aufheller (100 % Wirksubstanz, bezogen auf Warengewicht)
2 g/l Na-acetat
1 ml/l Essigsäure (100 %)
0,5 g/l Nonylphenolpolyglykolether (Nonylphenol + ca. 8 EO)

Die Anfangstemperatur betrug ca. 40°C, die Flotte wurde während etwa 30 Minuten auf 85°C aufgeheizt und es wurden weitere 30 Minuten bei dieser Temperatur aufgehellt. Am Ende der Abkühlphase des Apparates wurde die Ware intensiv heiß und kalt gespült und geschleudert. Die Trocknung erfolgte bei knapp 100°C.

Es wurde ein Weiß mit sehr guter Brillanz erhalten. Die Remissionsmessung am Remissionsspektralphotometer DMC 25 und die Berechnung des Weißgrades gemäß der Formel nach Ganz:
$Wg = (D\,Y) + (P\,x) + (Q\,Y) + C$
ergaben den vorzüglichen Weißgrad 254.

Beispiel 5

Im Labor-Baumfärbeapparat wurde einbadig mit Na-chlorit gebleicht und mit der Verbindung nach Beispiel 1 aufgehellt.
Material: 20 g Polyamid-6-Taft.
Flotte 1:20.
0,25 % Optischer Aufheller,
1,25 g/l Na-chlorit, 100 %
1,2 g/l Na-nitrat
1,2 g/l Kaliumhydrogentartrat,
0,5 g/l Nonylphenoloxethylat (Nonylphenol + ca. 8 EO)

Die Behandlung, insbesondere die Temperaturführung erfolgte wie unter Beispiel 4 beschrieben.

Es resultierte ein Weiß, welches die Aufhellung aus Beispiel 4 deutlich übertraf. Es wurde ein Weißgrad von 276 (Formel nach Ganz) gefunden.

Für weitere Beispiele wurde die Verbindung gemäß Beispiel 3 eingesetzt.

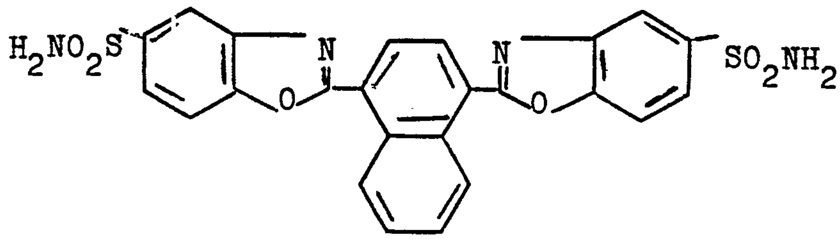

Die Verbindung ist nicht wasserlöslich, sie wurde wie folgt dispergiert:
0,100 g Substanz wurden siedend in 10 ml eines Gemisches aus DMF + Nonylphenoloxethylat (Nonylphenol + 23 EO) = 1 + 1 gelöst.

Die siedende Lösung wurde unter kräftigem Rühren quantitativ in ca. 70 ml Wasser eingetragen. Im Wasser waren zuvor 0,5 g teilverseifter Polyvinylalkohol gelöst worden. Nachdem die entstandene Dispersion unter Rühren auf Raumtemperatur abgekühlt war, wurde sie auf 100 ml aufgefüllt.

Beispiel 6

Labor-Baumfärbeapparat, Flotte 1:20, E-Wasser,
30 g Polyamid-6-Taft
30,0 ml der beschriebenen Dispersion (= 0,15 % Verb. gemäß Beispiel 3 (100 %) bez. auf Warengewicht)
3 g/l Na-Acetat
1 ml/l Essigsäure, 100 %
in 400 ml Flotte.

Es wurde wie in Beispiel 1 gearbeitet.

Dabei wurde ein Weißgrad von 249 erreicht (Formal nach Ganz).

Beispiel 7

Einbadige Na-chloritbleiche und Aufhellung gemäß Beispiel 5. Anstelle der dort genannten Verbindung wurde zur Aufhellung die Dispersion der Verbindung nach Beispiel 3 eingesetzt, wie oben beschrieben.
Labor-Baumfärbeapparat. 20 g Polyamid-Taft, Flotte 1:20, E-Wasser
30,0 ml Aufhellerdispersion (= 0,15 % Verb. gemäß Beispiel 3, 100 %, bezogen auf Warengewicht)
1,25 g/l Na-chlorit
1,2 g/l Na-nitrat
1,2 g/l anionisches Tensid
2,0 g/l Kaliumhydrogentartrat

Temperaturführung und Behandlung war wie in Beispiel 4 angegeben.

Auch diese Verbindung liefert in Gegenwart von Na-chlorit ein besseres Weiß. Es wurde ein Weißgrad von 266 gemessen (Formel nach Ganz). Der Weißgrad von Polyamid-6-Taft (Rohware) ist 77 (Formel nach Ganz).

## Ansprüche

1. Verbindungen der allgemeinen Formel (1),

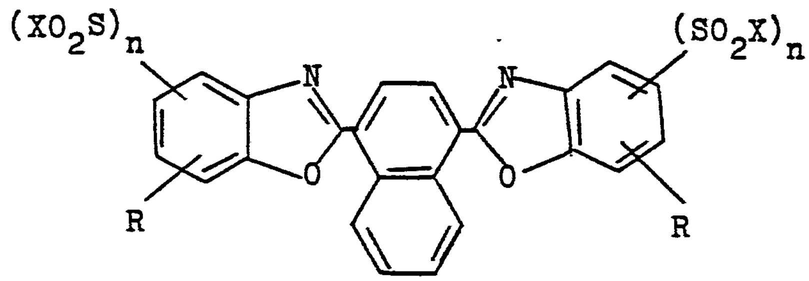

(1)

in welcher
n 1 oder 2,
X eine OMe-Gruppe,
Me ein Proton, ein Alkalimetallkation oder ein gegebenenfalls substituiertes Ammoniumkation,
R Wasserstoff, eine niedere Alkylgruppe oder Halogen und für den Fall, daß
n gleich 1 ist,
X auch eine $-NR^1R^2$-Gruppe, wobei $R^1$ und $R^2$ für Wasserstoff oder eine niedere Alkylgruppe steht, bedeuten.

2. Verbindungen der allgemeinen Formel (1), bei welchen
n gleich 1,
X eine OMe-oder $NH_2$-Gruppe,
Me ein Proton, ein Alkalimetallkation oder ein gegebenenfalls substituiertes Ammoniumkation und
R Wasserstoff bedeuten.

3. Verbindungen der Formel (2),

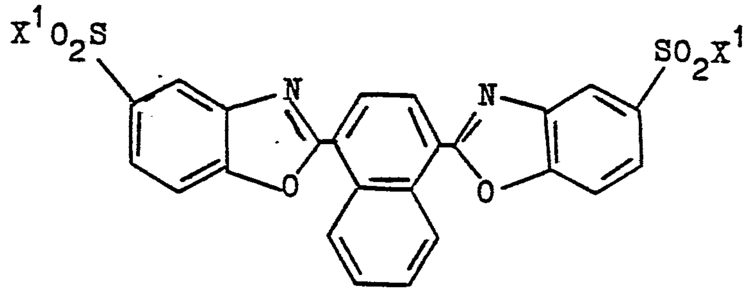

(2)

in welcher
X eine OMe-oder $NH_2$-Gruppe und
Me ein Proton, ein Alkalimetallkation oder ein gegebenenfalls substiuiertes Ammoniumkation, bedeuten.

4. Verfahren zur Herstellung der Verbindungen nach Anspruch 1, dadurch gekennzeichnet, daß man 2 Mol einer Verbindung der Formel

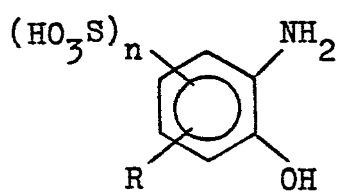

mit einem Mol 1,4-Naphthalin-dicarbonsäure kondensiert und gegebenenfalls die so erhaltene Verbindung der Formel

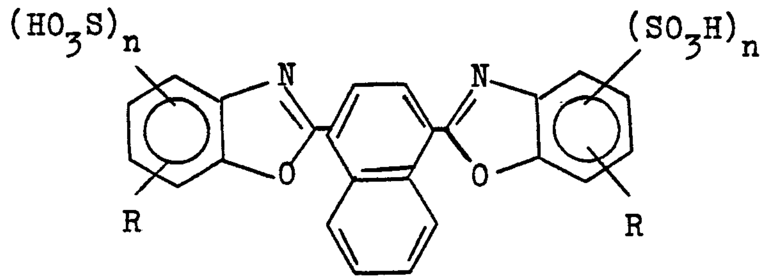

mit einem gegebenenfalls substituierten Amin neutralisiert und/oder mit einem Chlorierungsreagenz in die entsprechende Di-sulfochlorid-Verbindung und durch anschließende Umsetzung mit einem Amin der Formel
$HNR^1R^2$
in die Di-sulfonamid-Verbindung überführt.

5. Verfahren nach Anspruch 1, dadurch gekennzeichnet, daß man zur Herstellung solcher Verbindungen der For mel 1, worin X eine $-NR^1R^2$ Gruppe bedeutet, 2 Mol einer Verbindung der Formel

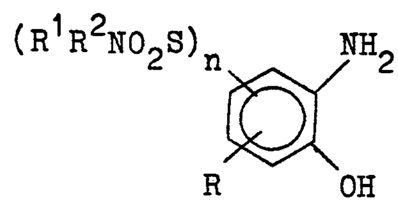

mit einem Mol 1,4-Naphthalin-dicarbonsäurechlorid kondensiert, gegebenenfalls unter Zwischenisolierung der als Zwischenstufe auftretenden Bis-acylamino-Verbindung.

6. Verwendung der Verbindungen nach Anspruch 1 als Optische Aufheller, insbesondere für Polyamid und Wolle.